# EUROPEAN PATENT SPECIFICATION

(11) **EP 3 075 397 B1**
(45) Date of publication and mention of the grant of the patent: **29.03.2017**
(21) Application number: 16460018.1
(22) Date of filing: 01.04.2016
(51) Int. Cl.: A61L 2/12, H05B 6/80, A23L 3/01, H05B 6/70

(54) **THE METHOD AND SYSTEM FOR MICROWAVE STERILISATION OF LIQUID MEDIA**
VERFAHREN UND SYSTEM FÜR MIKROWELLENSTERILISATION VON FLÜSSIGEN MEDIEN
PROCÉDÉ ET SYSTÈME DE STÉRILISATION PAR MICRO-ONDES DE MILIEUX LIQUIDES

(30) Priority: 03.04.2015 PL 41188315
(43) Date of publication of application: 05.10.2016
(73) Proprietor: Enbio Technology Spolka z o.o., 81-198 Suchy Dwor (PL)
(72) Inventor: Krajczynski, Marek, 81-326 Gdynia (PL)
(74) Representative: Kwapich, Anna

(56) References cited:
- US-A- 5 108 701
- US-A- 5 750 966
- US-A1- 2003 091 487
- US-A1- 2008 310 995

## Description

The invention concerns the method and system for sterilisation of liquid media. It can be used to sterilise different materials in the liquid or semi-solid phase, such as food samples, microbiological growth media, or buffer solutions, particularly in laboratory conditions.

Among different known sterilisation methods, systems, and devices there are methods, systems, and devices harnessing microwave energy. Microwave sterilisation is a thermal process similar to that which occurs in steam autoclaves, though the heat energy transfer is different. In steam autoclaves, heat energy is transferred convectionally through the vessel walls. In microwave autoclaves designated for sterilising liquids, on the other hand, the microwave energy is converted to heat directly in the liquid contained in the vessel placed in the process chamber in the area exposed to microwave radiation. Thanks to it the heating time, i.e. the time in which the process temperature required for the sterilisation of the material is reached, can be reduced substantially. The minimised time of heating and keeping the material in the process temperature is particularly important for retaining the stability of the sterilised media, since the shorter the exposition of the medium to high temperature, the lesser is the undesired decomposition of its components. Hence, the microwave methods and devices are today the most advantageous solutions for the sterilisation of liquids. The maximum temperature which can be achieved for water in the atmospheric pressure is 100°C, which in many cases is insufficient to attain the desired sterilisation of a liquid material. That is why the sterilisation processes are held under a raised pressure which enables achieving higher boiling temperature of the medium, and the sterilisation devices and systems are equipped with different elements which serve the purpose, such as compressors, or pneumatic system conduits with valves and pressure converters.

Known from patent publication US2008/0083749 is a solution which concerns the method and device for dehydrating and/or sterilising organic materials. The described method consists in placing the material in a microwave process chamber, bringing the pressure in the chamber to the desired value, exposing the material to microwave energy, and then removing water vapour from the chamber, where the material may be subject to pre-processing before it is placed in the chamber, and shaken during vapour discharge. In the presented solution, the device is composed of a microwave process chamber with a turntable plate fitted inside, and at least one microwave energy generator, where the chamber is connected to the vacuum system incorporating a pressure reducing pump. Presented are additional variants of the device appurtenances, in particular a rotating microwave-penetrable drum placed inside the chamber with a screw blade fitted to the drum's internal wall. The system may be fitted with a temperature sensor and a controller which switches off the microwave generator once the pre-set temperature is reached.

Disclosed in patent description US 5108701 is a method of quick sterilisation of biological materials which consists in exposing the material to microwave radiation in pressure increased in the process pressure chamber which is penetrable for microwave radiation, where the pressure and temperature is maintained at a specific level for a short time, usually for less than 10 or even 5 minutes, which is sufficient to achieve sterilisation. The chamber is designed to accommodate several containers with sterilised material at a time and may be subject to oscillation. The apparatuses used in the process are equipped with devices for controlling and setting the radiation and pressure in the process chamber so as to ensure the desired parameters of the sterilisation process. Presented are different variants of a preferable configuration of time, temperature and pressure of the sterilisation process, particularly for biological substances.

Known from patent description No. US 6579501 is an apparatus for carrying out wet chemical reactions, which ensures the highest possible effectiveness and reliability of chemical reactions thanks to the discharge of the reaction gas products so that they do not affect reaction stability or the pressure in the reaction chamber. The apparatus is fitted with a pressure chamber to accommodate a vessel with the reaction liquid, the bottom part of which is placed in the area exposed to the microwave resonator connected to the microwave energy generator with a waveguide. The apparatus is fitted with gas conduit units with valves, separate for the supply of pressurized gas from the compressor to the pressure chamber, and separate for the discharge of air from the pressure chamber. In addition, in the upper part of the pressure chamber there is a cooling element fixed to the lid which is inserted in the reaction vessel and connected to the conduits fitted with valves so as to supply and discharge the cooling liquid.

Also known is a microwave steriliser for the sterilisation of liquids, disclosed in the European patent application No. EP 2740494. It is composed of a body containing a process chamber with a tight lid, where the magnetrons fixed to the body serve as the source of microwave radiation. The steriliser is equipped with a pressure pneumatic system with a compressor, conduits, and valves, which enables increasing and reducing pressure in the process chamber. In addition, the steriliser is fitted with a hydraulic system composed of conduits, stub pipes for water supply and discharge, as well as supply, discharge, and water level control nozzles. The system enables the supply and removal of water to/from the process chamber, where the water forms a water coat encasing the vessel with the sterilised liquid, placed inside the process chamber.

The whole sterilisation process consists of three stages: the stage of increasing the pressure and heating up to the pre-set process temperature, the stage of maintaining the process pressure and temperature for a specified time, and finally the stage of cooling down and reducing the pressure so as to achieve conditions for safe opening of the process chamber and taking the sterilised medium out, in particular so as to avoid liquid overflow when opening the process chamber. An important criterion here is the shortest possible time of the whole sterilisation process while retaining the required conditions and parameters. The process is substantially shortened in those solutions in which the heating, i.e. the first stage, is achieved using microwave energy. The known liquid sterilisation methods and devices of this group focus on optimisation of stage two, i.e. the stage of sterilisation proper, in different conditions and requirements related to different applications and substances. None of the solutions known are intended to optimize stage three, i.e. the cooling down stage, which in most known solutions is very time consuming and consists in passive cooling of the sterilised material in the closed chamber once the heating is switched off. Also active cooling methods, by e.g. an exchange of the water coat surrounding the vessel with the sterilised liquid in the process chamber, prove ineffective because of the slow convection of heat between the liquid and its surrounding environment.

The method of microwave sterilisation of liquid media which consists in placing a vessel with the liquid medium in a pressure process chamber, heating the liquid medium up to the desired process temperature using microwave energy in pressure higher than atmospheric which eliminates overflowing and enables maintaining the process temperature for the time required for sterilisation, and then - after switching the source of microwave energy off - in cooling down and reducing the pressure to the level which enables opening the process chamber, according to this invention is characterized in that, in the cooling stage, the pressure in the process chamber is reduced at a controlled pace, which makes it possible to keep the liquid medium in constant boiling and evaporating without any overflows, until the pressure is equal to the ambient pressure.

Preferably, the pressure goes down at a pace which decreases in a non-linear manner over time.

The pressure reduction pace slows down in at least two phases of different speeds, where each of the subsequent phases lasts longer than the preceding phase.

Each subsequent phase lasts at least twice as long as the preceding phase, and the pressure reduction phase is at least twice slower than in the preceding phase.

The parameters and milestones marking individual phases are determined according to a pre-set algorithm based on pre-set values of: the volume of the liquid medium, process temperature, pressure, and duration of the liquid medium heating stage.

The pressure is regularly monitored in the cooling down stage, and the results are entered in the softwared processor in the control block. There, the data and the entered parameters of the liquid medium heating stage are processed using the pre-set algorithm, in effect of which an output control signal is generated to control the regulation discharge valve which is used to control the pressure reduction pace on an a continuous basis.

The system for microwave sterilisation in which the tight pressure chamber is connected to the source of microwave energy with a waveguide, where the chamber interior is connected to the compressor via a pressure converter, as well as to the regulation discharge valve and safety valve, and where the system is equipped with a feeder block, according to the invention is characterised in that the output of the pressure converter is connected to the input of the control block fitted with a softwared processor and connected to the data input module, and the outputs of the control block are connected to the compressor and the regulation discharge valve.

An advantage of the solution according to the invention is the optimisation of the process of reducing the pressure and temperature of the sterilised liquid medium which translates to the shortening of the cooling stage following the sterilisation proper and in consequence to reducing the total duration of the sterilisation process.

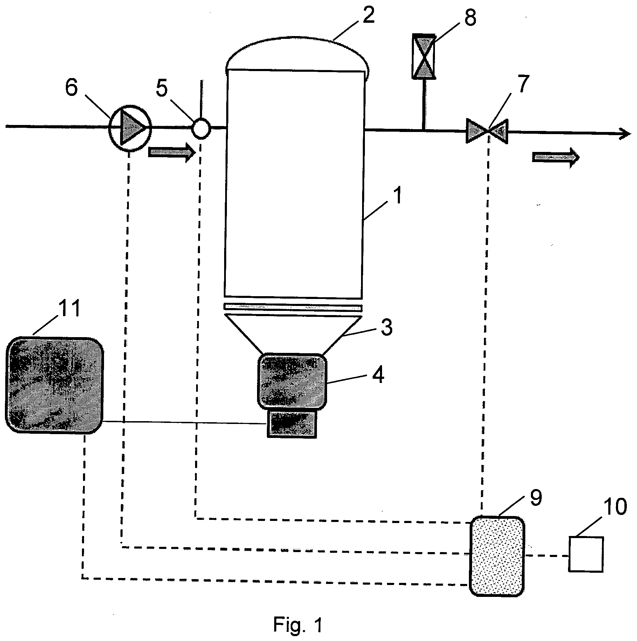

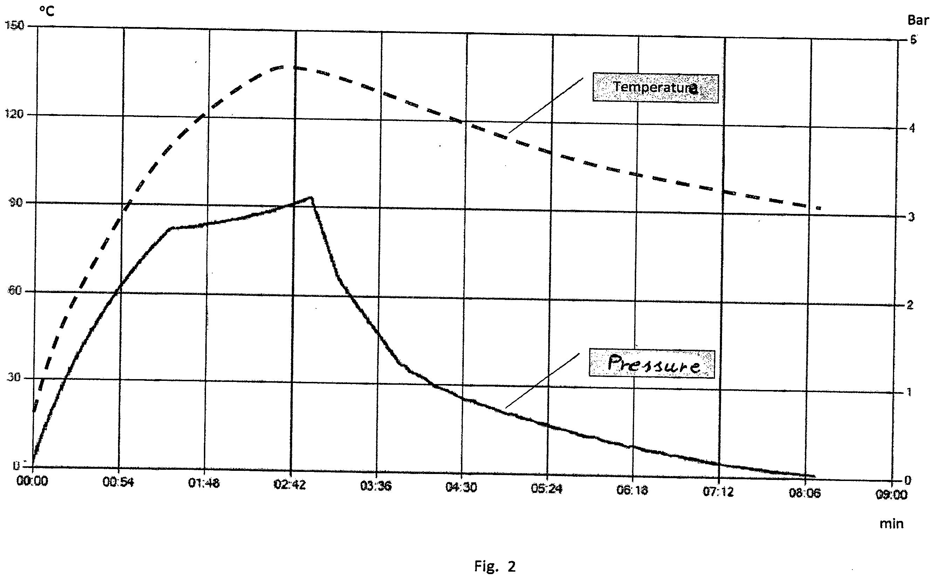

An exemplary embodiment of the invention is shown with a drawing, where fig. 1 shows the diagram of the system for microwave sterilisation of liquid media, and fig. 2 shows exemplary pressure and temperature curves for the method of microwave sterilisation of liquid media.

An exemplary method of microwave sterilisation of liquid media is embodied in a microwave autoclave system which contains a cylindrical process chamber 1 with a tight lid 2, a conical waveguide 3 and a magnetron serving as the source of microwave energy 4, pressure converter 5, compressor 6, regulation discharge valve 7 and safety valve 8. The system also includes a control block 9 equipped with a softwared processor, data input module 10 and feeder block 11.

The process of microwave sterilisation of liquid media in the exemplary embodiment follows the procedure described further on. Before use, the autoclave sterilisation system is calibrated so as to set the process parameters in individual phases for different volumes of the liquid medium subject to sterilisation. At this preliminary stage the system is additionally equipped with a temperature sensor which measures the current temperatures of the liquid medium. The results of the calibration measurements of pressure and temperature for different volumes of the medium, and the pre-set process time and process temperature of sterilisation enable determination of the parameters for individual stages in the process using appropriate calculation algorithms, where the parameters will guarantee the desired temperature of the sterilisation process and optimized cooling stage. Once the system has been calibrated, a vessel with the liquid medium which will be subject to sterilisation is placed inside the process chamber 1 of the microwave autoclave and the lid 2 of the process chamber 1 is closed. The volume of the liquid medium is entered in the data input module 10 and the device is switched on using the control block 9. This starts the compressor 6 which pumps air to the process chamber 1. The pressure is continuously measured using the pressure converter 5 and the measured value is entered in the control block 9 which starts the microwave energy source 4 once the pre-set pressure value is reached, where the energy source is used to heat the liquid medium in the process chamber 1. The overpressure in the process chamber prevents the boiling and overflowing of the liquid at the temperature required for its sterilisation. After specified time, depending on the volume of the medium, the control block 9 emits signals which turn off the compressor 6 and the source of microwave energy 4. Then, the cooling stage starts. In the method according to the invention the medium is cooled down by forcing its boiling and evaporation over the whole pressure reduction process in such a way so as to prevent liquid overflow. This is achieved by reducing the pressure in the process chamber 1 at a controlled pace using the regulation discharge valve 7 controlled with the signal emitted by the control block 9. The pressure reduction pace is controlled so that it decreases in a non-linear manner over time, with several increasingly longer phases clearly identifiable, where the pace decreases from phase to phase. The non-linear nature of transfer from phase to phase guarantees proper counteraction in the event of undesirable overheating, should it occur. The parameters of the cooling stage are determined according to the pre-set algorithm in the softwared processor of the control block 9, based on the entered data and the continuous pressure measurements. Once the pressure equals the ambient pressure the process completed, the lid can be lifted and the vessel with the sterilised liquid medium taken out. In the exemplary embodiment the liquid medium of 0.5 I in volume was heated to the temperature of 135°C under the relative pressure of 3 Bar. Once the heating was switched off, the cooling progressed in three phases. In phase I, which lasted approximately 0.5 minute, the pressure was reduced from the current value to 1.9 Bar at the pace of 30-50 mBar/sec; in phase II (ca. 1 minute) the pressure was reduced from 1.9 to 0.9 Bar at the pace of 8-20 mBar/sec; and in the longest 5-minute phase, i.e. phase III, the pace of brining the pressure to the ambient level was slowest (below 7 mBar/sec). The exemplary curves reflecting changes in the pressure and temperature over time are shown on fig. 2. The total duration of the whole sterilisation process, including the time of increasing pressure in the chamber, time of heating, and time of reducing the pressure and cooling, was approximately 8 minutes. In other methods known so far the process of sterilisation with passive cooling in the closed chamber and pressure which decreases spontaneously as the temperature goes down lasts several times longer.

The microwave autoclave system is composed of a cylindrical process chamber 1 with a tight lid 2, in its bottom part connected to the magnetron which serves as the source of microwave energy 4 with a conical waveguide 3. The process chamber 1 is connected to the compressor 6 via the pressure converter 5, and to the regulation discharge valve 7 and safety valve 8. The output of the pressure converter 5 is connected to the input of the control block 9, and the outputs of the control block 9 are connected to the compressor 6 and the regulation discharge valve 7. The control block 9 is fitted with a softwared processor and connected to the data input module 10. In addition, the system is equipped with a feeder block 11 which provides the necessary power supply to the system elements. The softwared processor of the control block 9 uses a pre-set algorithm to process the data entered as pre-set process parameters and the results of continuous pressure measurements in the process chamber (1), and generates output control signals for the compressor 6 and the regulation discharge valve 7.

The shortening of the total duration of the process by way of reducing the duration of the sterilised medium cooling stage is particularly important in laboratories where sterilisation of small samples is performed frequently and in large numbers.

## Claims

1. The method of microwave sterilisation of liquid media which consists in placing a vessel with the liquid medium in a pressure process chamber, heating the liquid medium using microwave energy to the desired process temperature in pressure higher than atmospheric, which eliminates overflowing of the liquid, and in maintaining the process temperature for the time required for sterilisation, and then - after switching the source of microwave energy off - in cooling down and reducing the pressure to the level which enables opening the process chamber, **characterized in that** the pressure in the process chamber in the cooling stage is reduced at a controlled pace, which makes it possible to keep the liquid medium in constant boiling and evaporating without any overflows, until the pressure is equal to the ambient pressure.

2. The method according to Claim 1, **characterized in that** the pressure goes down at a pace which decreases in a non-linear manner over time.

3. The method according to Claim 2, **characterised in that** the pressure reduction pace slows down in at least two phases of different speeds, where each of the subsequent phases lasts longer that then preceding phase.

4. The method according to Claim 3, **characterised in that** each subsequent phase lasts at least twice as long as the preceding phase, and the pressure reduction phase is at least twice slower than in the preceding phase.

5. The method according to Claim 4, **characterised in that** the parameters and milestones marking individual phases are determined according to a pre-set algorithm based on pre-set values of: the volume of the liquid medium, process temperature, pressure, and duration of the liquid medium heating stage.

6. The method according to Claim 5, **characterised in that** the pressure is regularly monitored in the cooling down stage, and the results are entered in the softwared processor in the control block (9); there, the data and the entered parameters of the liquid medium heating stage are processed using the pre-set algorithm, in effect of which an output control signal is generated to control the regulation discharge valve (8), which is used to control the pressure reduction pace on a continuous basis.

7. The system for microwave sterilisation in which a tight pressure chamber (1) is connected to a source of microwave energy (4) with a waveguide (3) where the chamber interior is connected to a compressor (6) via a pressure converter (5) as well as to a regulation discharge valve (7) and a safety valve (8) and where the system is equipped with a feeder block (11) **characterised in that** the output of the pressure converter (5) is connected to the input of a control block (9) fitted with a softwared processor and connected to a data input module (10), and the outputs of the control block (9) are connected to the compressor (6) and the regulation discharge valve (7).

## Patentansprüche

1. Verfahren für Mikrowellensterilisation der flüssigen Medien, wobei ein Behälter mit dem flüssigen Medium in einer Prozesskammer platziert wird, das flüssige Medium mittels der Mikrowellenenergie auf die erforderliche Prozesstemperatur erwärmt wird und zwar unter einem Druck, der höher ist als der Umgebungsluftdruck, sodass kein Überlaufen erfolgt, wobei die Prozesstemperatur über die zur Sterilisation erforderliche Zeit erhalten wird und nach dem Ausschalten der Mikrowellenenergiequelle eine Abkühlung und Drucksenkung erfolgt, sodass die Prozesskammer geöffnet werden kann, **dadurch gekennzeichnet, dass** auf der Stufe der Kühlung der Druck in der Prozesskammer mit einer kontrollierten Geschwindigkeit sinkt, welche die Erhaltung des flüssigen Mediums im Zustand des ständigen Siedens hält, bis der Umgebungsdruck erreicht wird.

2. Verfahren nach Anspruch 1, **dadurch gekennzeichnet, dass** der Druck mit der nicht linear sinkenden Geschwindigkeit sinkt.

3. Verfahren nach Anspruch 2, **dadurch gekennzeichnet, dass** die Geschwindigkeit der Drucksenkung sich in mindestens zwei Stufen mit unterschiedlichen Geschwindigkeiten ändert, wobei die Dauer der jeweils nächsten Stufe länger als die der vorherigen Stufe ist.

4. Verfahren nach Anspruch 3, **dadurch gekennzeichnet, dass** die Dauer der jeweils nächsten Stufe mindestens zweimal so lang ist als die der vorigen Stufe und die Geschwindigkeit der Drucksenkung maximal der Hälfte der Geschwindigkeit der vorigen Stufe entspricht.

5. Verfahren nach Anspruch 4, **dadurch gekennzeichnet, dass** die Parameter und Grenzen der einzelnen Stufen anhand eines bekannten Algorithmus für die vorgegebenen Werte des Volumens des flüssigen Mediums, der Prozesstemperatur, des Drucks und der Dauer der Stufe der Erwärmung des flüssigen Mediums ermittelt werden.

6. Verfahren nach Anspruch 5, **dadurch gekennzeichnet, dass** auf der Stufe der Kühlung ständige Druckmessungen durchgeführt werden, deren Ergebnisse in einen mit Software ausgerüsteten Prozessor des Steuerungsblocks (9) eingegeben werden, in welchem diese Daten zusammen mit den eingegebenen Parametern der Stufe der Erwärmung des flüssigen Mediums anhand eines vorgegebenen Algorithmus verarbeitet werden und so ein Ausgangssteuerungssignal für das Regel-Ablassventil (8) generiert wird, das der Druck mit der kontinuierlich geregelten Drucksenkungsgeschwindigkeit geregelt wird.

7. System zur Mikrowellensterilisation, dessen dichte Prozesskammer (1) über einen einen Lichtleiter (3) mit der Quelle der Mikrowellenenergie (4) verbunden ist, wobei der Innenraum über einen Druckaufnehmer (5) mit dem Kompressor (6), dem Regel-Ablassventil (7) und dem Sicherheitsventil (8) verbunden ist und wobei ein Versorgungsblock (11) vorgesehen ist, **dadurch gekennzeichnet, dass** der Ausgang des Druckaufnehmers (5) mit dem Ausgang des Steuerungsblocks (9) verbunden ist, der mit einem über die Software verfügenden Prozessor verbunden ist, der mit dem Dateneingabemodul (10) verbunden ist und die Ausgänge des Steuerungsblocks (9) mit dem Kompressor (6) und dem Regeln-Ablassventil (7) verbunden sind.

## Revendications

1. Le procédé de stérilisation par micro-ondes de milieux liquides qui consiste à placer un récipient avec le milieu liquide dans une chambre de procédé sous pression, à chauffer le milieu liquide en utilisant une énergie micro-ondes à la température du procédé souhaitée sous la pression plus élevée que la pression atmosphérique garantissant le manque de bouillonnement, et à maintenir dans la température du procédé pendant le temps nécessaire à la stérilisation, et après l'extinction de la source d'énergie micro-ondes le refroidissement et la réduction de la pression jusqu'au niveau permettant l'ouverture de la chambre de procédé, **caractérisé en ce qu'**à l'étape de refroidissement la pression dans la chambre de procédé est réduite à une vitesse contrôlée permettant de maintenir le milieu liquide en ébullition constante et évaporation sans bouillonnement jusqu'à atteindre la pression ambiante.

2. Le procédé selon la revendication 1, **caractérisé en ce que** la pression s'abaisse à une vitesse décroissante non linéairement dans le temps.

3. Le procédé selon la revendication 2, **caractérisé en ce que** la vitesse d'abaissement de la pression est réduite dans au moins deux phases ayant des vitesses différentes, la durée de chaque phase successive étant plus longue que celle de la phase précédente.

4. Le procédé selon la revendication 3, **caractérisé en ce que** la durée de chaque phase successive est au moins deux fois plus long, et la vitesse d'abaissement de la pression au moins deux fois plus faible que dans la phase précédente.

5. Le procédé selon la revendication 4, **caractérisé en ce que** les paramètres et les limites des différentes phases sont déterminées selon l'algorithme prédéterminé pour des valeurs de consigne du volume du milieu liquide, la température du procédé, la pression et la durée de l'étape de chauffage du milieu liquide.

6. Le procédé selon la revendication 5, **caractérisé en ce qu'**à l'étape de refroidissement sont effectuées en continu des mesures de pression, dont les résultats sont introduits au processeur programmé dans le bloc de commande (9), dans lequel ces données, ainsi que les paramètres saisis de l'étape de chauffage du milieu liquide sont traités selon un algorithme prédéterminé, et par conséquent est généré un signal de commande de départ pour une vanne de régulation de décharge (8) au moyen de laquelle la pression est réduite avec la vitesse de réduction de pression réglée au courant.

7. Le système pour la stérilisation par micro-ondes, dans lequel la chambre de procédé sous pression étanche (1) est reliée par l'intermédiaire d'un guide d'ondes (3) avec la source d'énergie micro-ondes (4), et son intérieur est relié par l'intermédiaire d'un transducteur de pression (5) avec un compresseur (6), et aussi avec la vanne de régulation de décharge (7) et la soupape de sécurité (8) et muni d'un bloc d'alimentation (11), **caractérisé en ce que** la sortie du transducteur de pression (5) est reliée à l'entrée du bloc de commande (9) muni d'un processeur programmé et connecté au module de saisie des données (10), et les sorties du bloc de commande (9) sont connectées au compresseur (6) et à la vanne de régulation de décharge (7).
